(19) Europäisches Patentamt
European Patent Office
Office européen des brevets

(11) Numéro de publication: **0 069 678**
**A2**

(12) # DEMANDE DE BREVET EUROPEEN

(21) Numéro de dépôt: 82401292.6

(22) Date de dépôt: 08.07.82

(51) Int. Cl.[3]: C 07 H 15/04
A 61 K 31/70, G 01 N 33/54
C 07 G 7/00

(30) Priorité: 08.07.81 FR 8113447

(43) Date de publication de la demande:
12.01.83 Bulletin 83/2

(84) Etats contractants désignés:
AT BE CH DE FR GB IT LI LU NL SE

(71) Demandeur: CHOAY S.A
48, Avenue Théophile-Gautier
F 75782 Paris Cédex 16(FR)

(72) Inventeur: Feizi, Ten
94, Brunswick Road
Ealing London W5(GB)

(72) Inventeur: Gooi, Hock Chye
1, Brookland Hill
London NW11 6DU(GB)

(72) Inventeur: Sinay, Pierre
5 rue Jacques Monod
F-45100 Orleans(FR)

(74) Mandataire: Peaucelle, Chantal et al,
Cabinet Plasseraud 84, rue d'Amsterdam
F-75009 Paris(FR)

(54) **Dérivés de 3-fucosyl-N-acétyl lactosamine, leur préparation et leurs applications biologiques.**

(57) L'invention concerne des dérivés de substitution du trisaccharide 3-fucosyl-N-acétyl lactosamine et les applications immunologiques de ses dérivés et du trisaccharide lui-même, notamment en tant que réactif de diagnostic et en thérapeutique.

EP 0 069 678 A2

Croydon Printing Company Ltd

Dérivés de 3-fucosyl-N-acétyl lactosamine, leur préparation et leurs applications biologiques.

---

L'invention est relative à des dérivés de 3-fucosyl-N-acétyl lactosamine, leur préparation et leurs applications biologiques.

Elle concerne plus particulièrement des dérivés de 3-fucosyl-N-acétyl lactosamine répondant à la séquence trisaccharidique suivante

Gal β 1 → 4 Glc NAc
↑ 1,3
Fuc α

dans laquelle Gal représente un motif galactopyranosyle, Glc NAc, un motif 2-acétamido,2-déoxy-glucopyranosyle (qui sera appelé ci-après glucosamine) et Fuc un motif fucopyranosyle.

La séquence de la 3-fucosyl-N-acétyl lactosamine a déjà été détectée chimiquement sur certaines glycoprotéines et sur certains glycolipides de tissus normaux ou tumoraux. Aucune information n'a cependant été donnée sur sa distribution dans les tissus.

Il avait été suggéré que cette structure, présente sur des érythrocytes, avait pu conduire, dans un cas particulier, à une réponse immunologique. Mais, il n'en résultait aucune information sur une détection possible de cette séquence oligosaccharidique, en tant qu'antigène, dans divers tissus.

Aucune corrélation n'avait donc été établie entre la présence de cette structure dans les tissus et la possibilité de la détecter en tant qu'antigène.

De plus, ces connaissances ne pouvaient amener à établir de corrélations entre la présence de cette structure dans les tissus et la présence d'anticorps vis-à-vis de cette structure durant le développement normal des tissus ou dans les états pathologiques.

En outre, dans les travaux antérieurs effectués dans ce domaine, la matière étudiée était constituée par des produits naturels d'extraction. Or, de tels produits

contiennent souvent des contaminants, avec des substances non déterminées, ce qui introduit des facteurs d'inexactitudes quant à la spécificité des observations immunologiques.

Il a été à présent découvert que la séquence 3-fucosyl-N-acétyl lactosamine en question ne peut être détectée comme antigène que dans certaines conditions. Ces travaux établissent donc que si cette structure peut être chimiquement détectable, elle ne s'exprime pas dans tous les cas en tant qu'antigène.

Les travaux qui ont conduit à la mise au point de l'invention se rapportent à l'étude d'un anticorps, appelé anti-SSEA 1, formé contre un carcinome de souris. L'intérêt pour cet anticorps résultait de sa reconnaissance d'un antigène embryonnaire (de structure inconnue) apparaissant à un stade du développement embryonnaire de la souris correspondant à huit cellules, ou antigène SSEA-1.

Il a été alors trouvé que, d'une manière inattendue, l'anticorps anti-SSEA 1 reconnaissait des structures comportant la séquence 3-fucosyl-N-acétyl lactosamine en question. La démonstration de la reconnaissance spécifique de cette structure par l'anticorps anti-SSEA 1 a été apportée en utilisant le trisaccharide obtenu par voie de synthèse, et par là de pureté et de spécificité élevées.

Il a pu être ainsi établi que l'antigénicité du SSEA-1 résultait de la séquence 3-fucosyl-N-acétyl lactosamine.

L'étude des conditions et des tissus dans lesquels la séquence en question se trouve non seulement présente, mais où son antigénicité peut s'exprimer a permis de réaliser une autre étape de l'invention en conduisant à étudier l'utilité de la 3-fucosyl-N-acétyl lactosamine et de dérivés de substitution de ce trisaccharide, ainsi que des anticorps formés par le trisaccharide et ses produits de substitution conjugués, en particulier pour des diagnostics et en thérapeutique.

L'invention a donc pour objet de fournir de nouveaux dérivés du trisaccharide 3-fucosyl-N-acétyl lactosamine, utilisables comme réactifs biologiques ou facilitant grâce à

leurs caractéristiques propres la mise à profit des propriétés du trisaccharide en question. Elle vise également à fournir des voies d'accès à ces trisaccharides.

Elle a également pour but les applications, comme déterminant antigénique, dutrisaccharide 3-fucosyl-N-acétyl lactosamine et de ses dérivés, en particulier, les réactifs antigéniques et les anticorps élaborés à partir de ces produits ainsi que les immunoabsorbants résultant du couplage de ces produits sur des supports solides.

Les nouveaux trisaccharides de l'invention sont des dérivés de substitution du trisaccharide O-α-L-fucopyranosyl-(1→3)-[O-β-D-galactopyranosyl-(1→4)-2-acétamido-2-déoxy-D-glucopyranose] (ce trisaccharide est désigné dans la description et les revendications par le terme 3-fucosyl-N-acétyl lactosamine) et répondent à la formule :

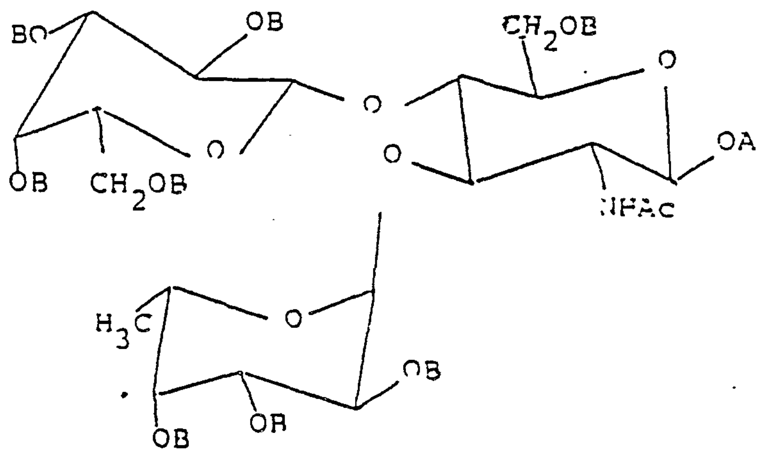

dans laquelle :

A représente

- un groupe alcoyle comportant 2 à 10 atomes de carbone, ou
- un groupe hydroxy-alcoyle avec 2 à 10 atomes de carbone comportant un ou plusieurs groupes hydroxyle, avantageusement un groupe hydroxyle en bout de chaîne, ou
- un groupe comportant une ou plusieurs liaisons insaturées, en particulier au moins un radical éthyléniquement insaturé, choisi parmi les radicaux alcényle, et plus spécialement les radicaux alcényle à 2 à 10 atomes de carbone, ou encore
- l'un des groupes précédents, mais comportant un ou plusieurs groupes éther, notamment deux ou encore trois, et/ou amine intercalaires,
- les différents groupes représentés par A pouvant être terminés par un groupement azoté notamment amino, amido,

hydrazine, azido, ou un groupement acide carboxylique ou un dérivé d'un tel groupement, notamment un éther ou un ester,

- les substituants B, identiques ou différents les uns des autres, représentent, le cas échéant avec un substituant voisin, des groupes de protection de radicaux hydroxyle et sont choisis parmi des groupes stables, non réactifs dans les conditions habituelles de synthèse osidique et facilement éliminables dans des conditions douces, compatibles avec le maintien de la structure osidique, en particulier par des groupes benzyle, benzoyle, benzylidényle ou acétyle, ou représentent un atome d'hydrogène.

Dans un groupe préféré de dérivés d'osides selon l'invention, A représente un radical alcoyle comportant 2 à 10 atomes de carbone, substitué par au moins un groupe hydroxyle, avantageusement en bout de chaîne, notamment un radical alcoylèneglycol ou hydroxyalcoyle ou encore un radical carboxylique ou un dérivé d'un tel radical.

Des produits de ce type, notamment ceux dans lesquels A représente un groupe $\alpha,\beta$-dihydroxypropyle, $\beta$-hydroxyéthyle ou $\gamma$-hydroxypropyle constituent des réactifs biologiques spécialement intéressants dans les applications biologiques faisant intervenir leur fixation sur une protéine ou un support insoluble comportant des fonctions amino libres, par exemple, pour la constitution respectivement d'antigènes artificiels et d'immunoabsorbants.

D'autres produits également intéressants à cet égard comportent en bout de chaîne un groupe carboxylique ou un dérivé et un tel groupe tel qu'un éther ou un ester.

Dans un autre groupe préféré de trisaccharides selon l'invention, A comporte en bout de chaîne un groupe azoté, notamment un groupe amino, amido, azido ou hydrazino.

Un autre groupe préféré de trisaccharides comprend, le cas échéant en plus d'au moins l'une des dispositions qui précèdent, une ou plusieurs fonctions éther intercalaires, notamment deux ou encore trois fonctions éther.

Un autre groupe préféré encore comprend comme chaîne A, un groupement alcoylène, terminé par un groupement

fonctionnel.

Des trisaccharides préférés des groupes évoqués ci-dessus renferment une chaîne A répondant aux structures (1), (2) ou (3) suivantes :

(1) : $-(CH_2)_n - O - (CH_2)_{n'} - O - (CH_2)_m - R$

(2) : $-(CH_2)_{n''} - O - (CH_2)_{m'} - R$

(3) : $-(CH_2)_{n'''} - R$

dans lesquelles -n, n' et m, identiques ou différents les uns des autres, sont égaux à 1, 2, 3, 4 ou 5, m pouvant en outre être égal à 0, la somme de n + n' et éventuellement m étant avantageusement un nombre de 4 à 10, de préférence égal à 6 ;

-n" et m', identiques ou différents l'un de l'autre, sont égaux à 1, 2, 3, 4 ou 5, m' pouvant être égal en outre à 0, n" ou éventuellement n" + m' étant avantageusement un nombre de 4 à 10, de préférence égal à 6 ou 8, et

-n''' est un nombre de 1 à 10, avantageusement de 4 à 10, de préférence égal à 8, et

-R représente un groupement fonctionnel.

Dans des chaînes A de produits préférés de l'invention, R représente un groupe azoté tel que $-NH_2$, $-N_3$ ou $-NHNH_2$.

Dans d'autres chaînes de structure (1) ou (2), R représente un atome d'hydrogène.

D'autres chaînes préférées de structure (1), (2) ou (3) ci-dessus renferment un radical R représentant un groupe -COOR' ou -COR', R' représentant un atome d'hydrogène ou un radical alcoyle avec 1, 2 ou 3 atomes de carbone ou encore R représente un groupe $-CONH_2$.

D'autres trisaccharides préférés comportent dans une chaîne A telle que définie ci-dessus avec un ou plusieurs groupements amine intercalaires.

Des trisaccharides également avantageux notamment en tant qu'intermédiaires pour l'obtention des trisaccharides désirés, correspondent à des produits dans lesquels A présente la structure (1), (2) ou (3) ci-dessus, dans laquelle

$\underline{R}$ représente un groupe alcényle avec 2 à 10 atomes de carbone, de préférence un groupe allyle.

La préparation de ces nouveaux trisaccharides s'intègre avantageusement dans le processus de synthèse du trisaccharide 3-fucosyl-N-acétyl lactosamine décrit par JACQUINET et SINAY dans J. Chem. Soc. p 314-318, 1979.

On rappelle que, dans son schéma le plus général, ce procédé comporte, dans une première étape, la réaction du bromure de 2,3,4,6-tétra-O-acétyl-β-D-galactopyranosyle avec du benzyl-2-acétamido-3-O-allyl-6-O-benzyl-2-déoxy-α-D-glucopyranoside dans du dichloroéthane-1,2, en présence de bromure de mercure et de tamis moléculaire 4 Å. Le produit résultant est alors constitué par le benzyl 2-acétamido-4-O (2,3,4,6-tétra-O-acétyl-β-D-galactopyranosyle)-3-O-allyl-6-O-benzyl-2-déoxy-α-D-glucopyranoside. Dans une deuxième étape, le groupe allyle est éliminé avec du chloro -(tristriphénylphosphine)-thodium et le produit obtenu est condensé avec du bromure de 2,3,4-tri-O-benzyl-α-L-fucopyranosyle. Les groupes acétyle sont ensuite éliminés et le trisaccharide 3-fucosyl lactosamine est obtenu après hydrogénolyse catalytique.

Dans le cadre de l'invention, ce schéma est donc avantageusement utilisé, mais on le réalise en mettant en oeuvre un motif glucopyranose, élaboré de manière à comporter le bras de substitution désiré.

Selon un mode de réalisation de ces produits permettant plus spécialement l'élaboration de chaînes (1), c'est-à-dire comportant au moins deux groupements éther intercalaires, on met en oeuvre, aux fins de constitution de ces chaînes, du diéthylèneglycol ou du triéthylèneglycol.

Cette variante présente l'avantage de permettre l'utilisation d'un produit de faible coût et dont la réactivité offre l'accès à de nombreuses substitutions.

Les chaînes élaborées sont alors condensées avec un dérivé réactif de la glucosamine évoquée en rapport avec le procédé de synthèse de JACQUINET et SINAY ci-dessus, par exemple, le chlorure de cette glucosamine, ou une glucosamine dans laquelle toutes les positions, excepté la position 4, sont bloquées. Le disaccharide résultant est mis en oeuvre dans le procédé en question où il est condensé avec un dérivé réactif de fucosyle.

Dans une autre variante, qui permet plus spécialement l'obtention de trisaccharides substitués sur le motif glucosamine par une chaîne de structure (3), on a avantageusement recours aux méthodes classiques de type KOENIGSKNORR comportant la réaction d'un dérivé réactif d'une β-glucosamine, tel qu'une 1,2-oxaline, avec un acide monocarboxylique de structure :

$$HO-(CH_2)_{n'''}-R$$

dans laquelle <u>n'''</u> et <u>R</u> présentent les significations données ci-dessus en rapport avec la chaîne (3).

La β-glucosamine de formule :

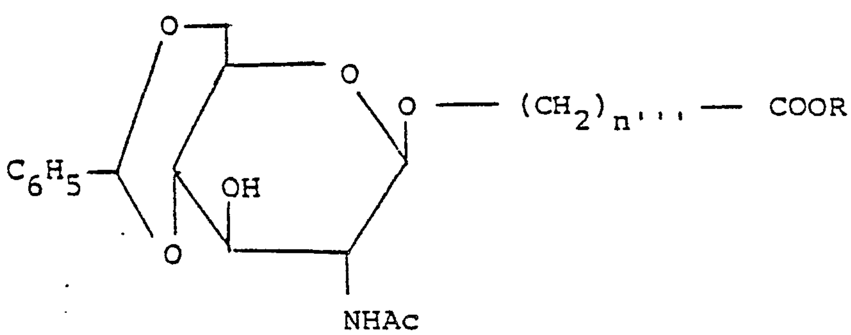

est ensuite intégrée dans le procédé de JACQUINET et SINAY.

Selon un autre mode de réalisation permettant d'obtenir les trisaccharides de l'invention dans lesquels A représente un groupe allyle ou une chaîne pouvant être obtenue en utilisant la réactivité du groupe allyle, on procède tout d'abord à la synthèse d'un trisaccharide répondant à la structure des trisaccharides de l'invention, dans lequel tous les groupes -OH sont engagés dans des groupements protecteurs, et sont avantageusement benzylés. Selon les réactions classiques de la synthèse organique, on introduit un groupement allyle en position 1, en $\beta$, de la glucosamine.

A partir de l'allyl-1-$\beta$-glucosamine, grâce à la réactivité du groupe allyle, il est possible de procéder à l'allongement de la chaîne de manière à introduire les différents groupes souhaités, L'articulation des différentes réactions impliquées s'effectue selon les techniques classiques de synthèse.

Il est notamment avantageux, pour allonger la chaîne allylique, de soumettre le dérivé allyle à une réaction d'hydroboration, en utilisant un réactif tel que le 9-borabicyclo [3.3.1]nonane, puis à l'action d'une base.

On obtient alors une chaîne correspondante terminée par un groupement fonctionnel alcool. On notera que ce groupe peut être aisément éthérifié et permet l'introduction d'un groupe éther intercalaire dans la chaîne de substitution A, les autres hydroxyle du trisaccharide étant protégés par des éthers benzyliques.

En vue des applications où il apparait souhaitable de disposer d'une chaîne servant de bras de couplage pour la fixation sur un support, on traite la chaîne à groupement alcool précédente par un halogénure d'allyle pour introduire un nouveau groupement allyle qui sera à son tour avantageusement soumis à une réaction d'hydroboration.

Ce jeu de réactions permet d'augmenter, comme souhaité, la longueur de la chaîne de substitution et de disposer en bout de chaîne de groupements fonctionnels permettant par leur réactivité une meilleure mise à profit des propriétés des osides de l'invention.

Parmi les diverses possibilités de traitement offertes, il est avantageux de recourir à une tosylation puis de faire réagir le dérivé tosylé avec un azide.

Comme indiqué ci-dessus, les travaux qui sont à la base de la mise au point de l'invention ont montré que la séquence 3-fucosyl-N-acétyl lactosamine pouvait être détectée en tant qu'antigène et qu'elle ne s'exprimait que dans certaines conditions et dans certains tissus où la densité de cette séquence était relativement élevée. Une série de résultats d'essais radioimmunologiques effectués à cet égard sont donnés ci-après à titre illustratif.

L'invention fournit alors des moyens permettant d'assurer la détection de cette séquence et de mettre à profit cette détection dans diverses applications immunologiques de grand intérêt.

L'invention vise, en particulier, des réactifs de diagnostic, mettant à profit le caractère antigénique du trisaccharide 3-fucosyl-N-acétyl lactosamine, ou de ses dérivés de substitution définis ci-dessus. Ces réactifs sont utilisables tels quels ou sous forme immobilisée. Ils peuvent également être utilisés pour obtenir des anticorps spécifiques. Les anticorps rentrent également dans le cadre de l'invention.

Ces réactifs antigéniques constituent des composés de référence et s'avèrent spécialement précieux pour la caractérisation de structures et d'anticorps. En particulier, ils permettent de détecter la présence ou d'étudier les variations de taux d'anticorps dans diverses conditions liées au développement cellulaire (embryogénèse) ou à des états pathologiques qui correspondent alors à un stade où le caractère antigénique de la séquence 3-fucosyl-N-acétyl lactosamine a pu s'exprimer.

Ils présentent notamment un grand intérêt pour la détection d'anticorps libérés au cours de maladies auto-immunes, de troubles de la grossesse (notamment dans le cas de toxémies, éclampsies) et chez les patients atteints de certains cancers ou encore dans le cas de tératocarcinomes.

On mesure à cet égard l'intérêt de pouvoir disposer aisément de produits de pureté élevée grâce à l'obtention possible de la 3-fucosyl-N-acétyl lactosamine et de ses dérivés par voie de synthèse. Ces réactifs antigéniques de pureté élevée sont ainsi avantageusement utilisables pour détecter et/ou étudier des anticorps polyclônaux ou monoclônaux.

Comme l'ont montré les travaux exposés par H. Koprowski aux Federation Meetings à New-Orleans en Avril 1982, (dont question dans Proceeding Abstracts index, 3rd volume, Guinzburg V. et Koprowski H.), la séquence 3-fucosyl-N-acétyl lactosamine est par exemple présente dans des tumeurs gastro-intestinales. On mesure donc l'intérêt de pouvoir détecter de telles tumeurs avec les produits purs de l'invention. Ces travaux, en plus de ceux des inventeurs décrits ci-dessus, montrent l'étendue de l'utilisation de ces produits.

L'invention fournit donc des réactifs biologiques utilisables dans un large champ d'application. Leur production par voie de synthèse facilite leur élaboration sous une forme appropriée à une utilisation donnée. Grâce à l'invention, on dispose ainsi de toute une gamme de réactifs pour les diverses applications immunologiques de ces produits.

L'invention vise également les anticorps spécifiques formés vis-à-vis du trisaccharide 3-fucosyl-N-acétyl lactosamine et de ses dérivés de substitution selon l'invention et possèdent donc une pureté élevée.

D'une manière avantageuse, ces anticorps spécifiques permettent de déterminer comment l'antigénicité de la structure trisaccharidique en question peut être affectée durant le développement cellulaire. D'une manière générale, ils permettent également de vérifier si cette structure s'exprime ou non comme antigène dans les tissus.

De tels produits, qui seront avantageusement radio-marqués, soit par des radioisotopes-soit par des substances fluorescentes ou autres substances biochimiquement décelables pour faciliter les localisations, constituent de

précieux composés de référence en permettant de reconnaître la structure 3-fucosyl-N-a-étyl lactosamine en tant qu'antigène dans des séquences complexes de matériaux biologiques.

La 3-fucosyl-N-acétyl lactosamine et les dérivés selon l'invention sont également avantageusement mis en oeuvre, comme composés de référence, pour élaborer des substrats d'enzyme, en particulier, des substrats de glycosidases et de glycosyl-transférases.

Les chaînes de substitution définies ci-dessus permettent la mise à profit des propriétés antigéniques du trisaccharide 3-fucosyl-N-acétyl lactosamine dans des antigènes artificiels ou dans des immunoabsorbants. Ces chaînes constituent en effet de précieux bras espaceurs permettant, selon leur nature, une fixation sur des protéines ou divers types de supports insolubles aux fins d'élaboration respectivement d'antigènes artificiels et d'immunoabsorbants.

L'invention vise donc également, en tant que produits nouveaux, ces antigènes artificiels et ces immunoabsorbants.

Pour l'élaboration d'antigènes artificiels, on utilise des trisaccharides dont les chaînes se terminent plus spécialement par un groupe -COOH ou $-NH_2$ le couplage étant effectué sur une macromolécule porteuse, pouvant être administrée à l'animal.

Les chaînes possédant au moins une fonction éther intercalaire, c'est-à-dire au sein de la chaîne, et avantageusement deux ou trois, présentent un caractère hydrophile précieux.

Les chaînes de ce type terminées par un groupe amino sont particulièrement avantageuses pour le couplage du trisaccharide sur des supports solides de grande stabilité, comportant des sites de fixation -OH ou -COOH, ou encore $-CONH_2$.

Des chaînes terminées par un groupe -OH, -COOH ou un dérivé de ces groupes sont avantageusement fixées sur des macromolécules comme celles choisies dans le groupe comprenant la poly-L-alanine-lysine, l'albumine, l'albumine

de sérum bovin, la thyroglobuline ou la polylysine.

Les chaînes de substitution des trisaccharides de l'invention permettent ainsi d'accéder à des supports conduisant à des immunoabsorbants dotés d'une capacité d'absorption d'anticorps anti-3 fucosyl lactosamine élevée et ne donnant pas lieu à des absorptions non spécifiques.

Des supports avantageusement utilisables sont à base de polymères renfermant des polysaccharides notamment à base de cellulose, de l'agarose ou de l'agarose réticulée (notamment le produit commercialisé sous la marque Sépharose par Pharmacia).

D'autres supports appropriés comprennent la silice, des billes de verre ou de latex ou des polymères mixtes renfermant d'autres dérivés polymères tels que des polyacrylamides. D'autres supports de ce type convenant dans le cadre de l'invention, on peut citer ceux commercialisés sous les marques ULTROGEL ou encore MAGNOGEL par IBF (Industrie Biologique Française).

D'une manière générale, afin d'empêcher toute fixation non spécifique, le produit de couplage est avantageusement mis en suspension dans une solution permettant de saturer les sites de fixations encore libres.

Cette mise en suspension est, par exemple, effectuée dans une solution protéique.

Pour le couplage desproduits de l'invention sur les supports du type évoqué ci-dessus, on a avantageusement recours aux méthodes générales utilisées en chromatographie d'affinité.

Selon les techniques courantes, le support solide sur lequel on souhaite immobiliser le dérivé d'oside est au préalable activé. Cette activation peut être réalisée selon différents moyens, par exemple, à l'aide de bromure de cyanogène, de carbonyldiimidazole, d'hydrazine ou de glutaraldéhyde ou tout autre produit connu à cet effet et équivalent.

L'invention fournit donc des immunoabsorbants de synthèse particulièrement précieux, permettant l'élaboration de sérums-tests à spécificité 3-fucosyl-lactosamine ou dépourvus d'anticorps anti-3-fucosyl-lactosamine qu'on désire éliminer.

L'intérêt de ces immunoabsorbants de l'invention est d'autant plus grand qu'ils sont régénérables. Ainsi, après élution en milieu acide des anti-3-fucosyl-N-acétyl lactosamine fixés sur des immunoabsorbants et concentration, on élimine les anticorps anti-3-fucosyl lactosamine et l'on dispose des immunoabsorbants qui peuvent être à nouveau utilisés. Les anti-3-fucosyl-N-acétyl lactosamine sont alors récupérés avec des titres élevés.

D'autres caractéristiques et avantages de l'invention apparaîtront dans les exemples qui suivent et en se reportant aux figures.

EXEMPLE 1 : Préparation du trisaccharide 8-azido-3,6-dioxa octyl 2-acétamido-3-O-/α-L-fucopyranosyl/-4-O/β-D-galactopyranosyl/-2-désoxy-β-D-glucopyranoside (composé a).

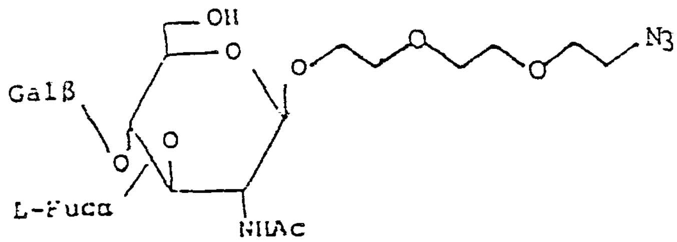

dans laquelle Gal et Fuc présentent les significations données ci-dessus et Ac un groupe acétyle.

On opère selon le procédé de JACQUINET et SINAY décrit dans J.Chem. Soc. p. 314-318, 1979, mais à la place de benzyl glycoside (α), on utilise un β-glycoside avec

$HO-(CH_2)_2-O-(CH_2)_2-O-(CH_2)_2N_3$

Cet azide est obtenu à l'aide des trois étapes :

a) monotosylation du triéthylèneglycol

b) formation du dérivé azido correspondant

c) condensation avec le chlorure de 2-acétamido-3,4,6-tri-O-acétyl-2-désoxy-α-D-glucopyranosyle.

On réalise chacune de ces étapes en procédant comme suit :

a) monotosylation du triéthylèneglycol :

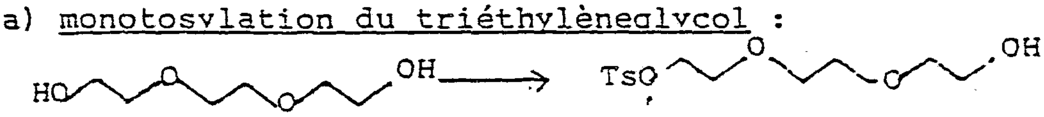

où Ts représente le groupe tosyle.

On dissout 1 g de triétgylèneglycol dans 10 ml de pyridine anhydre, on refroidit à 0°C puis on verse goutte à goutte une solution de 1,26 g de chlorure de tosyle (c'est-à-dire un équivalent) dans 5 ml de pyridine et on soumet le mélange à agitation pendant 4 heures. De l'eau glacée est alors ajoutée, puis on soumet le mélange résultant à agitation pendant une heure. Le traitement classique (extraction à l'aide de chloroforme, lavage de la phase chloroformique à l'eau et séchage avec du sulfate de sodium) conduit à un résidu qui est purifié par chromatographie sur colonne de gel de silice.

Rendement 60 à 75% en monotosyle de triéthylèneglycol.

b) passage au dérivé azido correspondant :

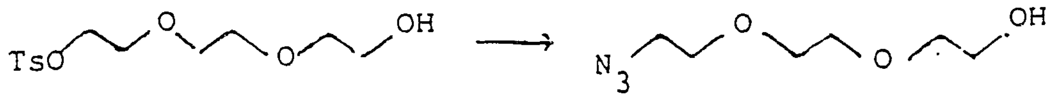

On dissout 20 g de dérivé monotosyle dans 20 ml de DMF anhydre, 8,5 $NaN_3$ (2 équivalents par rapport au dérivé monotosyle), on soumet le mélange à agitation pendant 1 heure à 80°C, puis on refroidit. On élimine ensuite les sels insolubles par filtration sur verre fritté. Après plusieurs lavages au chloroforme, on récupère le filtrat global (y compris les eaux de lavage avec le chloroforme) et on le lave avec de l'eau jusqu'à pH neutre.
La phase organique est séchée sur sulfate de sodium et évaporée sous vide. Le DMF est éliminé sous 10 mm de mercure entre 80 et 120°C, puis le produit, séché, est distillé sous 10 mm de mercure à environ 150°C. On obtient ainsi, dans cette manipulation environ 7,5 g de produit, ce qui représente un rendement de 65% en azide. Une distillation supplémentaire sous $10^{-3}$ mm de mercure conduit à un produit ayant un point d'ébullition de 60°C.

Analyse élémentaire

| | calculé | trouvé |
|---|---|---|
| Carbone | 41,13 | 41,34 |
| Hydrogène | 4,48 | 7,54 |
| Azote | 23,99 | 23,61 |

c) <u>condensation avec le chlorure de 2-acétamido-3,4,6-tri-O-acétyl-2-désoxy-α-D-glucopyranosyle</u> :

On fait réagir 12,5 g d'acétochloro N-acétylglucosamine, 3 g du dérivé d'azido précédent, 3 g de tamis moléculaire 4 Å, 2 ml de tétraméthylène, 20 ml de dichlorométhane anhydre, et l'on soumet le mélange à agitation à 0°C pendant 30 mn. On ajoute alors 7 g de triflate d'argent, puis on soumet ce mélange à agitation à l'abri de la lumière et de l'humidité pendant 17 heures à température ambiante.
Après avoir effectué le traitement habituel, (dilution avec de l'eau, extraction à l'aide de chloroforme, lavage de la phase chloroformique avec de l'eau et séchage de la phase chloroformique avec du sulfate de sodium), on soumet le

résidu obtenu à une chromatographie sur colonne de gel de silice de 250 g en utilisant com me éluant l'éther-méthanol (20 : 1 volume de méthanol). On récupère 6,5 g de produit cristallin.

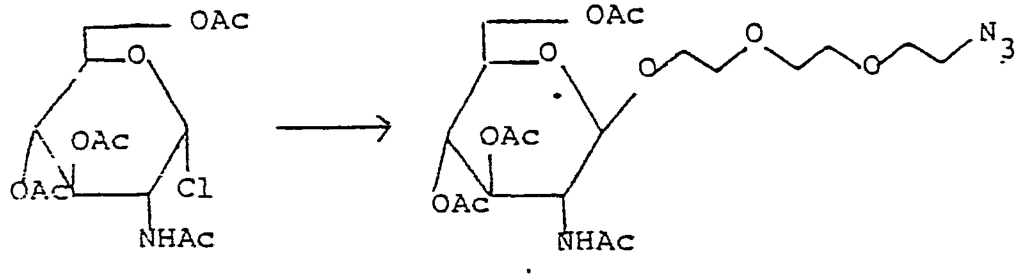

Rendement de 75%

On récupère 1,3 g supplémentaire de produit en traitant les produits restant à la surface de la colonne, ce qui correspond à un rendement total de 90%.

Les caractéristiques physiques et analytiques de ce produit sont les suivantes :

PF = 69-70°C

$[\alpha]_D^{20°C}$ = - 29,6° (c = 1,08 ,chloroforme)

Analyse élémentaire :

| | calculé | trouvé |
|---|---|---|
| Carbone | 47,61 | 47,48 |
| Hydrogène | 6,39 | 6,17 |
| Azote | 11,10 | 10,89 |

Au cours d'une étape supplémentaire, on procéde à l'élimination des groupes acétyle en opérant comme suit : 500 mg du produit tétraacétylé sont à dissoudre dans 10 ml de méthanol anhydre, on ajotue quelques gouttes d'une solution de méthoxyde de sodium dans le méthanol et on soumet le mélange à agitation pendant 2 heures à température ambiante Le traitement habituel évoqué ci-dessus est suivi d'une purification sur gel de silice. On récupère 285 mg de produit.

Rendement 75%.

L'analyse élémentaire du produit purifié est la suivante :

| | calculé | trouvé |
|---|---|---|
| Carbone | 44,44 | 44,65 |
| Hydrogène | 6,93 | 7,00 |
| Azote | 14,81 | 14,65 |

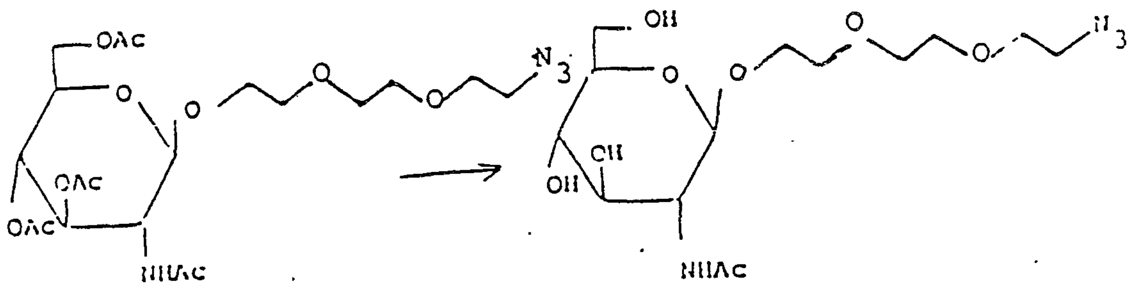

Le produit "désacétylé" peut être utilisé tel quel, c'est-à-dire sans purification préalable pour former le dérivé :

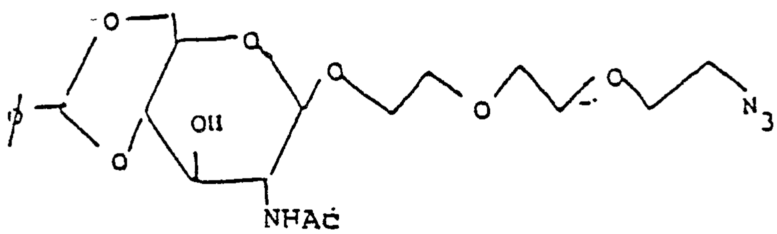

On dissout 300 mg du produit désacétylé dans 6 ml de DMF anhydre, on ajoute 133 mg de diméthoxy toluène, une quantité catalytique d'acide paratoluène sulfonique, et on laisse le réacteur sur un évaporateur de type Büchi dont le bain-marie est maintenu à 55-60°C pendant une heure. On refroidit ensuite le mélange réactionnel, on le dilue avec du chloroforme et on le lave avec une solution de bicarbonate de sodium puis avec une solution de chlorure de sodium. Après séchage de la phase organique sur du sulfate de sodium et évaporation, on obtient un résidu que l'on recristallise dans l'éthanol, ce qui conduit à 304 mg (rendement de 82%) d'un produit cristallin présentant les caractéristiques suivantes :

PF : 193-194°C

$[\alpha]_D^{20°C}$ : 76,5° (c = 1,62 chloroforme)

Analyse élémentaire

| | Calculé | Trouvé |
|---|---|---|
| Carbone | 54,07 | 54,24 |
| Hydrogène | 6,48 | 6,34 |
| Azote | 12,01 | 12,14 |

EXEMPLE 2 : Préparation du trisaccharide 8-amino-3,6-dioxa octyl-2-acétamido-3-O-[α-L-fucopyranosyl]-4-O-[β-D-galactopyranosyl]-2- désoxy-β-D-glucopyranoside (composé b).

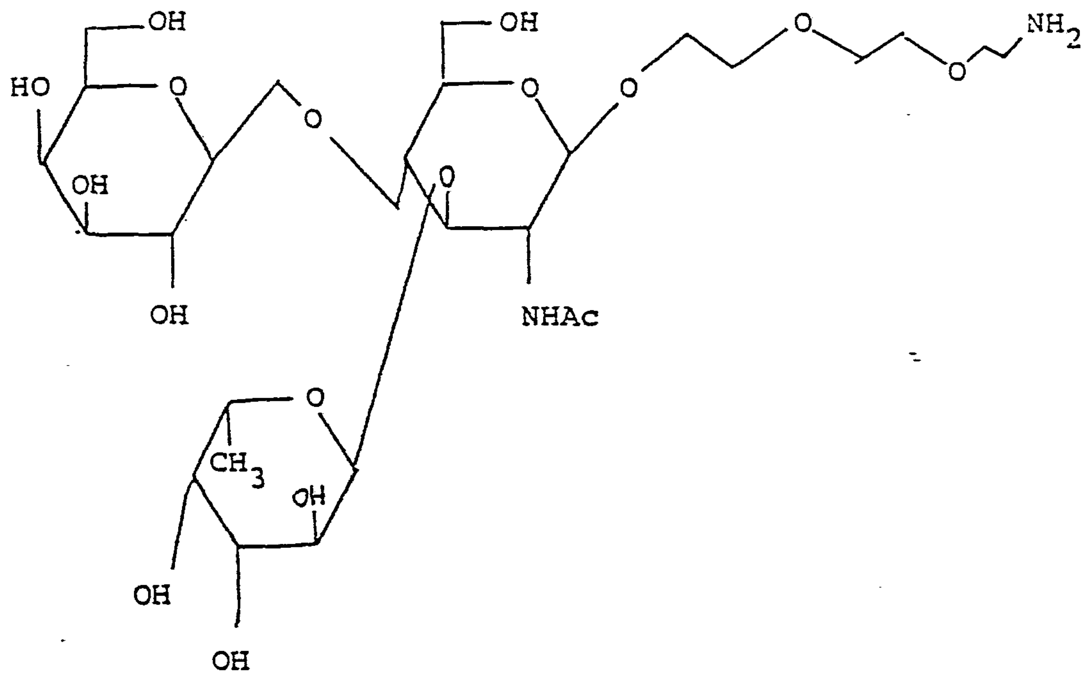

On soumet le dérivé azido obtenu selon l'exemple 1 à l'action de méthylate de sodium, en présence de méthanol, puis on hydrogène en présence de Pd/C et de $CH_3COOH$, ce qui conduit au produit recherché.

EXEMPLE 3 : Préparation du trisaccharide répondant à la formule donnée dans l'exemple 2, mais dans laquelle la chaîne de substitution est constituée par le groupe $-O-(CH_2)_8-COOCH_3$, à savoir du 8-méthoxy carbonyloctyl 3,6-dioxa 2-acétamido 3-O [α-L-fucopyranosyl]-4-O-[β-D-galactopyranosyl]-2-désoxy-β-D-glucopyranoside (composé c). Ce produit est obtenu en soumettant le trisaccharide de formule d.

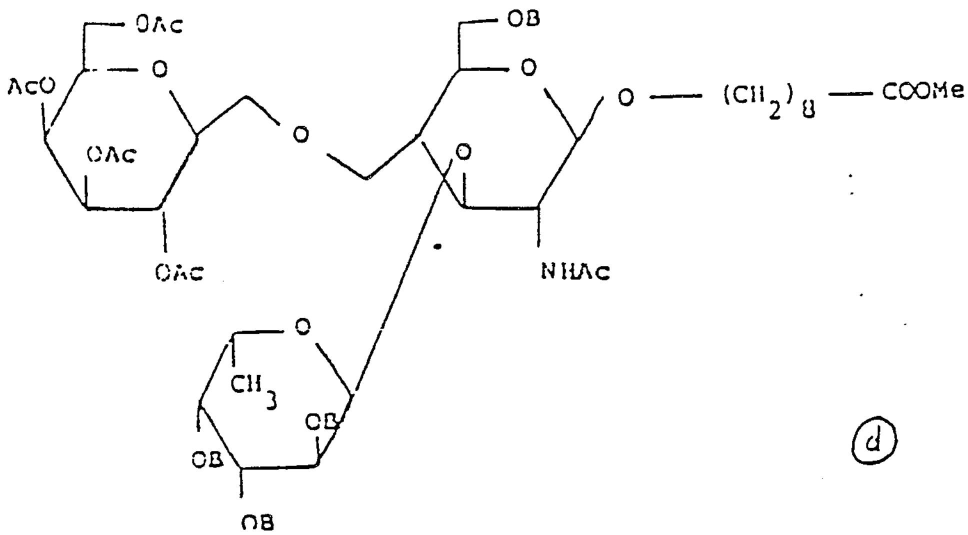

(d)

au traitement donné dans l'exemple 2.

<u>EXEMPLE 4</u> : Préparation du trisaccharide 7 méthoxy carbonyl 3,6 dioxa heptyl 2 acétamido 3-O /α L-fucopyranosyl/ 4-O/β D.galactopyranosyl/ 2 désoxy-β-D-glucopyranoside (composé <u>17</u>) de formule :

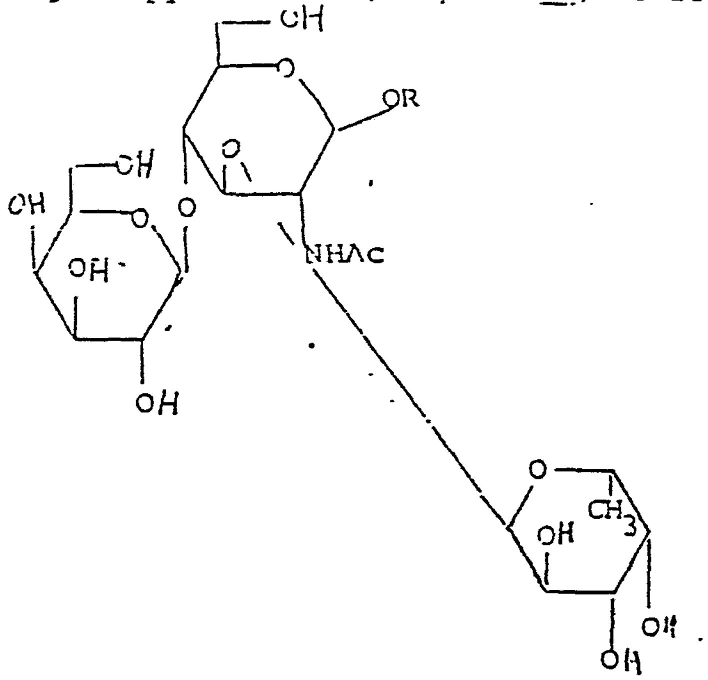

Cette synthèse est effectuée selon les étapes a) à p) suivantes:

a) -- <u>préparation du 7-phényl 3,6 dioxa heptanol</u> (composé <u>2</u>) selon le schéma suivant :

$HO-(CH_2)_2-O(CH_2)_2-OH \longrightarrow HO-(CH_2)_2-O-(CH_2)_2-OBn$ (2)

Dans un ballon, sous agitation mécanique, on mélange 90 g de KOH en poudre et 500 ml de dioxanne. On chauffe à 90°C puis on ajoute 90 ml de diéthylèneglycol dilué avec 100 ml de dioxanne, puis 108 ml de chlorure de benzyle. Au bout d'une demi-heure, le mélange devient épais. On ajoute 100 ml de dioxanne. La réaction est suivie en chromatographie sur couche mince (ccm) ($CHCl_3$ - MeOH :9/0,5) On ajoute 40 ml d'eau et on poursuit l'agitation pendant une demi-heure. On effectue ensuite une concentration, une extraction à l'aide d'acétate d'éthyle puis un lavage à l'eau. /on extrait alors le monobenzyle et les traces de dibenzyle/. On concentre la phase organique. Le sirop obtenu est dissous dans un mélange hexane/eau. (Le dibenzyle passe dans l'hexane). On ajoute à la phase aqueuse du chloroforme pour extraire le monobenzyle. La phase organique est séchée par du sulfate de sodium, filtrée, concentrée et distillée sous vide /5 mm Hg/ Pt éb : 110-120°C On récupère 105,6 g (rendement 63,46%).

b) - préparation du 9-phényl-2,5, 8 trioxa 1-méthoxycarbonyl nonyl (composé 3), selon le schéma suivant :

$BnO-(CH_2)_2-O-(CH_2)_2-OH$ ⟶ $BnO-(CH_2)_2-O-(CH_2)_2-O-CH_2-COOMe$

(2) (3)

A 34,34 g de monobenzyle (0,175 mole), on ajoute 270 ml de DMF anhydre et 9,66 g de NaH 50% (1/1 équivalent) déshuilé au toluene.
Après agitation pendant 1 heure à -10°C sous argon, on ajoute goutte à goutte (pendant 1 heure), 40,15 g (22ml) de bromo-acétate de méthyle. (1,5 équivalent).
La réaction est terminée après une heure.
(ccm avec $CH_2Cl_2$/acétone : 4/1).
On ajoute 40 ml de méthanol et 5 ml d'acide acétique.
Après agitation pendant 1 heure, on concentre, puis on extrait à l'aide de dichlorométhane. La phase organique est séchée avec du sulfate de sodium, filtrée et concentrée.
On distille sous vide le liquide obtenu. Pt Eb = 130-150°C /5 mm $H_g$/. On obtient 24,7 g de produit (rdt = 52,6%).
Le spectre de RMN comporte les signaux suivants ($CCl_4$).
$\delta$ = 3,56 - 3,62 m 8 H ($CH_2-CH_2$) ; 3,65 s (3H) $OCH_3$ ; 4,03 s (2H) $O-CH_2CO$ ; 4,50 s (2H) $CH_2\phi$ ; 7,23 m (5H) $\phi$ aromatique.

c) - préparation du 7 carboxyméthyl 3,6 dioxa heptanol (composé 4) selon le schéma

$BnO-(CH_2)_2-O(CH_2)_2-O-CH_2-COOMe$ ⟶ $HO-(CH_2)_2-O-(CH_2)_2-O-CH_2COOMe$

(3) (4)

10 g de produit de départ sont dissous dans 100 ml de méthanol anhydre. On ajoute le catalyseur : Pd/C 10%.
On agite une journée sous pression d'hydrogène. La réaction est suivie en ccm éther-méthanol 10/1. On filtre la solution, puis on concentre. Le liquide obtenu est distillé sous vide 5 mm Hg. Pt/éb = 120-130°C. On obtient 5,9 g de produit (rdt = 90%).
Le spectre de RMN comporte les signaux suivants ($CCl_4$) : $\delta$ = 3,10 s (1 H) OH ; 3,56 - 3,62 m (8 H) $CH_2-CH_2$ ; 3,65 s (3H) $OCH_3$ ; 4,05 s (2H) $O-CH_2CO$.

d)- préparation du 7 méthoxycarbonyl (3,6 dioxa) heptyl-2 acétamido-3,4,6 tri-O-acétyl-2-désoxy β-D-glucopyranoside (composé 5) selon le schéma suivant :

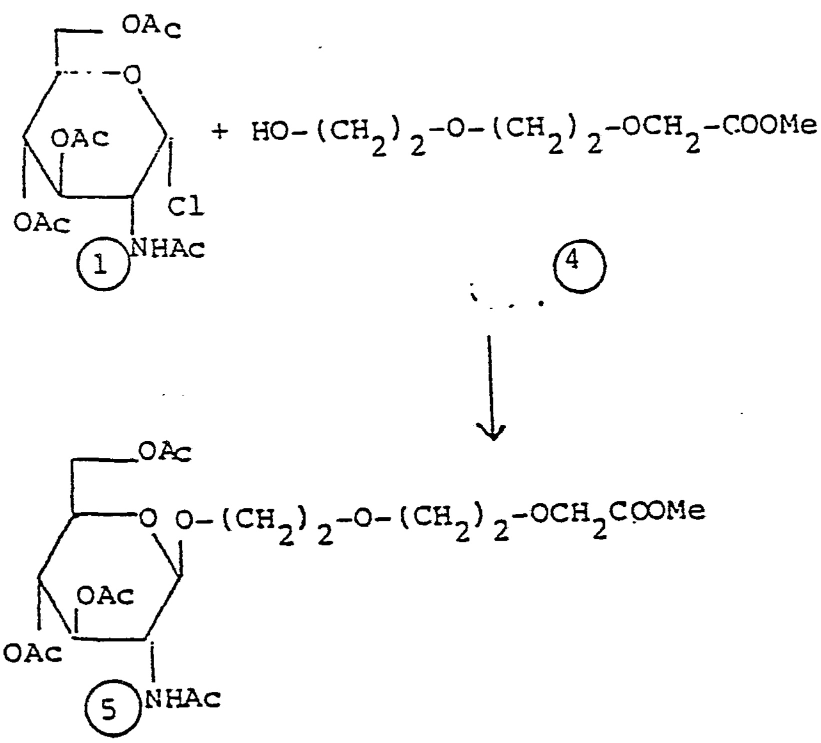


Le chlorure de 2-acétamido - 3,4,6 tri - O-acétyl 2 désoxy α -D-glucopyranosyle (composé 1) est préparé selon la méthode de D. MORTON dans Meth. in Carbohydrate Chem. VI p. 282.

A l'abri de la lumière et sous argon, on agite 1 g. de composé 4, 2,5 g. de composé 1, 1 g de $CaSO_4$ pulvérisé, séché à l'étuve à 400°C pendant 24 h, et 10 ml de $CH_2Cl_2$ distillé sur $P_2O_5$.

Le mélange est agité vigoureusement pendant 1 heure à température ordinaire.

Puis on ajoute 3 g de $HgCN_2$ (M = 252,6 g) finement broyé et séché pendant 24 h à 50°C sous vide. On laisse sous agitation durant 30 h à température ambiante, puis on dilue au chloroforme, on filtre sur Celite 545, on lave à l'aide de bicarbonate de sodium, d'iodure de potassium 10% puis d'eau. La phase organique est séchée avec du sulfate de sodium. Après filtration, on concentre la phase organique, puis le sirop obtenu est chromatographié [100 g de gel de silice :

éluant : éther - méthanol : 10/0,5/

On obtient un sirop in ore 1,9 g (rdt : 67,8%)

$[\alpha]_D^{20}$ = -30,6 [c = 1,11 $CHCl_3$]

Le spectre de RMN comporte les signaux suivants ($CDCl_3$) : $\delta$ = 1,8 - 2,3 (12H) $CH_3CO$ N-$CH_3$ ; 3,5-4,5 m (18 H) bras + $H_2$ $H_5$ $H_6$ $H_{6'}$; 4,95 - 5,12 m (3H) $H_1$ $H_3$ $H_4$; 6,90d (1 H) NH; $J_{NH,H_2}$ = 9Hz.

e) - préparation du 7-méthoxycarbonyl 3, 6 dioxa heptyl, 2 acétamido 2-désoxy-β-D-glucopyranoside (composé 6) de formule

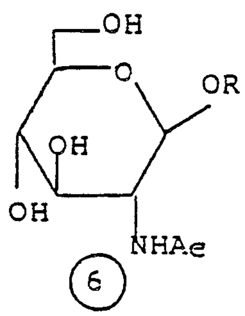

6

dans laquelle R représente la chaîne $-O(CH_2)_2-O-(CH_2)_2-O-CH_2-COOMe$.

On dissout 1 g de produit de départ dans 10 ml de méthanol absolu. On ajoute quelques gouttes d'une solution 2 M de méthylate de sodium. On soumet le mélange à agitation durant 2 h à température ambiante. La réaction est suivie en ccm $CHCl_3$ = MeOH 7/3.

Après 2 h, on ajoute de la résine H+ sèche /lavée avec du méthanol jusqu'à pH neutre). On filtre, puis on concentre. Le sirop obtenu est chromatographié (colonne de gel de silice : 30 g de gel de silice, éluant $CHCl_3$-MeOH 7/3).

On obtient 650 mg d'une mousse blanche (rdt : 86,5%).

$[\alpha]_D^{20}$ = - 21,6 (c = 0,6 MeOH)

f) - préparation du 7-méthoxycarbonyl 3,6 dioxa heptyl 2 acétamido 4,6 -O-benzylidène 2-désoxy β -D-glucopyranoside (composé 7) de formule :

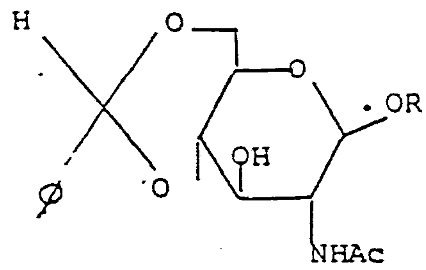

7

dans laquelle Ø représente le groupe $-C_6H_5$.
On se réfère à la méthode de M.E. EVANS dans Carbohydrate Research 21 (1972) 473.

On dissout 600 mg de produit de départ (M= 381 g) dans 5 ml de DMF anhydre . On ajoute 263 mg d'α,α diméthoxytoluène (M = 152 g) et une quantité catalytique d'acide paratoluène sulfonique. La réaction s'effectue à l'évaporateur rotatif à 55-60°C pendant 1 heure.

On suit la réaction en ccm ($CHCH_3$ - MeOH : 10/1).
On extrait au chloroforme et on lave la phase organique à l'eau jusqu'à pH neutre puis on concentre.
Après recristallisation dans l'éthanol, on récupère 620,4 mg (rdt = 84%) de P.F. 162°C ; $[\alpha]_D^{20}$ = - 69° (c = 1, $CHCl_3$).

Le spectre de RMN comporte les signaux suivants $CDCl_3$
δ : 2,02 s (3H) $N-CH_3$ ; 3,5-3,75 m (11H) $CH_2-CH_2$ ; $OCH_3$ ; 4,15 s (2 H) $O-CH_2-CO$ ; 4,20 - 4,50 m (2H) ; 4,82 d (1H) $H_1$ ; $J_{1,2}$ = 8 Hz ; 5,54 s (1H) CH-Ø ; 7,00 d NH ; $J_{NH,H_2}$ = 6Hz ; 7,2-7,6 m aromatique.

g) - préparation 7-méthoxycarbonyl 3,6-dioxaheptyl 2-acétamido 3-O-benzyl-4,6-O-benzylidène 2-désoxy β-D-glucopyranoside (composé 8) de formule :

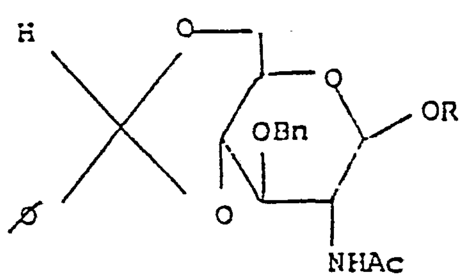

8

On agite vigoureusement à l'abri de l'humidité pendant 1/2 heure :

500 mg de produit de départ, 980 mg de BaO, 270 mg de Ba $(OH)_2$, $8H_2O$, 5 ml de DMF anhydre, puis on ajoute 0,2 ml de bromure de benzyle.

On soumet le mélange à agitation pendant 3 heures. La réaction est suivie en ccm : $CHCl_3$-MeOH 10/1. On constate l'hydrolyse du méthyl ester. On ajoute 5 ml de MeOH .. Après agitation pendant 1/2 heure, on concentre puis on extrait au chloroforme. On lave à l'eau la phase organique, celle-ci est ensuite séchée avec du sulfate de sodium, filtrée puis concentrée. Le résidu cristallin est dissous dans du méthanol anhydre. On ajoute une solution de diazométhane dans l'éther jusqu'à persistance de la coloration jaune. On obtient un produit plus migrant que le produit de départ. On concentre après 1 heure.

Apris cristallisation dans l'éthanol on récupère 435 mg deproduit, rdt = 73%, de P.F. = 182-184°C ; $[\alpha]_D^{20} = -16,6°$ (c = 1,02; $CHCl_3$) .

Le spectre de RMN comporte les caractéristiques suivantes : ($CDCl_3$) : $\delta$ = 1,92 s (3H) N-Ac ; 3,55-3,90 m (11H) $CH_2CH_2$, $OCH_3$; 4,00 s (2H) $OCH_2CO$ ; 4,20-5,00 m $H_3$, $H_4$, $H_1CH_2\phi$ ; 5,56 s (1H) $CH\phi$; 6,80 d NH ; $J_{NH,H2}$ = 8Hz ; 7,20-7,50 m (10H) aromatique.

h) - preparation du 7-méthoxycarbonyl 3,6 dioxaheptyl 2 acétamido 3-O-benzyl 2-désoxy-β-D-glucopyranoside (composé 9) de formule :

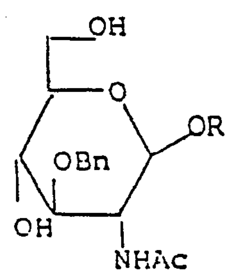

9

869 mg de produit de départ et 30 ml $CH_3COOH$ 75% sont chauffés à 70°C pendant 1 heure. La réaction est suivie en ccm ($CHCl_3$-MeOH:9/1). On concentre ensuite la solution puis on effectue une coévaporation avec du toluène. Le sirop obtenu est chromatographié sur colonne de gel de silice 25 g (éluant $CHCl_3$-MeOH;9/1). On obtient 660 mg d'un sirop incolore (rdt : 90%).
/cristallisation dans un mélange méthanol-éther, cristaux très hygroscopiques. Le sirop est utilisé tel quel/
$[\alpha]_D^{20} = -20,72°$ (c = 2,3; $CHCl_3$)
Le spectre de RMN comporte les signaux suivants : ($CDCl_3$) : $\delta$ 1,92 s (3 H) N-Ac : 2,82 s (2 H) OH ; 3,5-3,72 m $OCH_3$ - $CH_2$-$CH_2$; 4,08 -O-$CH_2$CO. 4,7-4,8 m 3H $H_1$ $\varphi$ $CH_2$; 6,54 d (1H) NH; $J_{NH,H2}$ = 8 Hz ; 7,3 s (5H) aromatique.

i) <u>préparation du 7-méthoxycarbonyl 3,6 dioxaheptyl 2-acétamido 6-O-benzoyl 3-O-benzyl-2-désoxy-β-D-glucopyranoside</u> (composé <u>10</u>) de formule

(10)

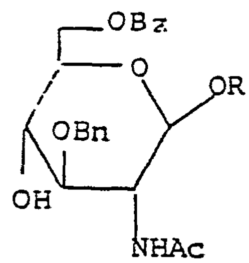

dans laquelle Bz représente un groupe benzoyle.
660 mg deproduit de départ sont dissous dans 15 ml dichlorométhane /distillé sur $P_2O_5$/.
On y ajoute 300 mg de cyanure de benzoyle et 3,5 ml de pyridine anhydre.
On soumet le mélange à agitation à l'abri de l'humidité
La réaction est suivie en ccm ($CHCl_3$-MeOH:9,5/0,5). En fin de réaction, on ajoute du méthanol anhydre (5ml). Après agitation pendant 2 heures, on concentre, on effectue une coévaporation de la pyridine avec du toluène. On obtient des cristaux blancs. Recristallisation dans du dichlorométhane-éther (605,2 mg ; rendement : 75%).

$[\alpha]_D^{20}$ = -17,5° (c = 1, $CHCl_3$) ; PF = 124-125°C.

Spectre de RMN ($CDCl_3$) :

$\delta$ = 1,95 (3 H) N-Ac : 3,50-3,80 m (14 H) dont $O-CH_2-CH_2-O$, $OCH_3$ ; 4,09 (2H) $OCH_2CO$ ; 4,60 s (2H) ; 4,75 (3H) ; 6,50 d (1H) NH ; 7,20-7,60 (8H) benzyl, benzoate, méta, para ; 8,10 d (2H) benzoate (ortho).

j) - préparation du 1-O-trichloroacétimidyl 2,3,4,6 - tétra -O -acétyl $\alpha$ -D-galacto-pyranoside (composé 11) de formule

(11)

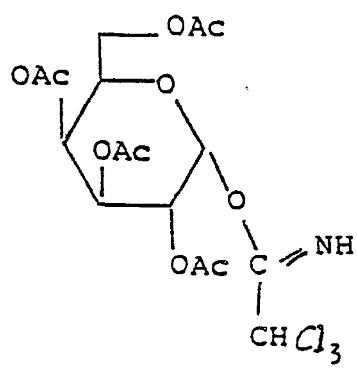

2,4 g de tétra-O-acétyl galactose, préparés selon R.R. SCHMIDT and J. MICHEL Ang. Chem. Int ed (19) (1980) 731-32, F. CRAMER, H. PAWELZIK, H.J. BALDAUF. Ber. (1958) 1049-54), sont dissous dans 20 ml de dichlorométhane anhydre. On ajoute 250 mg de sodium, puis 7 ml de trichloroacétonitrile. On soumet à agitation à température ambiante à l'abri de l'humidité pendant 5 heures. La réaction est suivie en ccm (hexane-acétate d'éthyle 9/5). Le mélange est filtré, puis concentré. Le sirop obtenu est chromatographié sur une colonne de gel de silice (150 g de gel) (éluant : hexane-acétate d'éthyle 9/5). On récupère 1,3 g du produit le plus migrant (rendement 38,4%). P.F. = 122-123°C. $[\alpha]_D^{20}$ = + 115,5 (c = 1,6 $CHCl_3$) et 884 mg du produit le moins migrant (rendement 26,1%). RMN ($CDCl_3$) du dérivé : $\delta$ = 2,05-2,20 2d (12H) OAc ; 4-4,26 m (2H) $H_6H_{6'}$ ; 4,3-4,6 m (1H) $H_5$ ; 5,4 m (2 H) $H_3H_4$ ; 5,55 q (1 H) $H_2$ ; $J_{1,2}$ = 4Hz ; $J_{2,3}$ = 2 Hz : 6,60 d (1H) $H_1$ ; $J_{1,2}$ = 4HZ ; 8,65 s (1H) NH.

k) - préparation du 7-méthoxycarbonyl 3,6 dioxaheptyl-2-acétamido 6-O-benzoyl 3-O-benzyl - 4-O-/2,3,4,6 tétra-O-acétyl-β D galactopyranosyl/ 2-désoxy-β.D.glucopyranoside (composé 12) selon le schéma suivant :

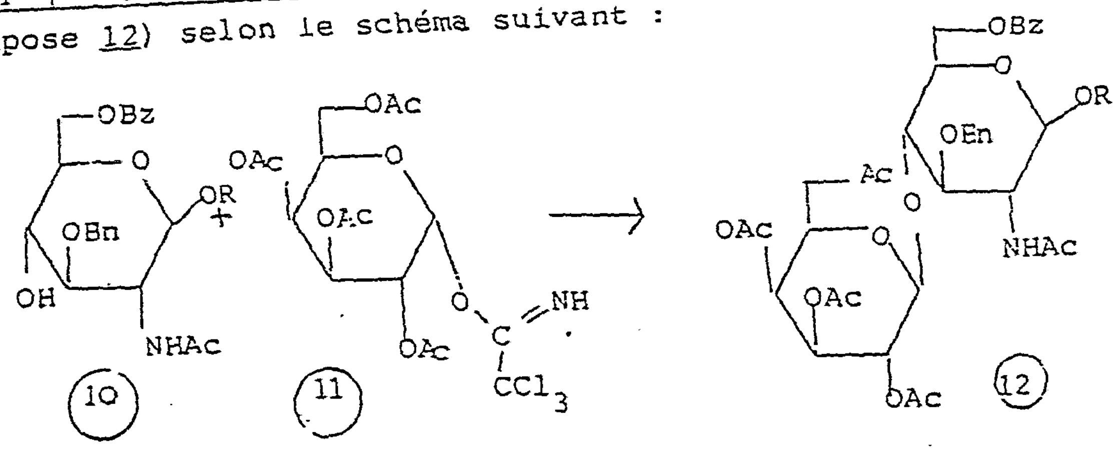

On dissout : 435 mg d'aglycone (composé 10) et 600 mg d'imidate (composé 11) dans 2 ml de dichlorométhane anhydre.

On ajoute 8 gouttes d'éthérate de trifluorure de bore.

On soumet le mélange à agitation à l'abri de l'humidité à température ordinaire pendant 7 heures. Pour compléter la réaction, on ajoute 260 mg d'imidate et après agitation pendant une nuit, on extrait au dichlorométhane. On lave la phase organique au bicarbonate de sodium puis à l'eau. Celle-ci est ensuite séchée par du sulfate de sodium puis filtrée et concentrée. Le sirop obtenu est chromatographié sur une colonne de gel de silice /55 g, éluant: $CHCl_3$ - Acétone 7/3/.

On récupère 475,5 mg d'une fraction pure $[\alpha]_D^{20} = -6,9$ (c = 1,07; $CHCl_3$)

- produit de départ 65,2 mg

rendement par rapport à l'aglycone qui a réagi: 81 %.

RMN $CDCl_3$ : δ = 2,00 s - 2,12 s - 2,16 s (15 H) N-Ac. $COCH_3$ ; 3,50 - 4,06 m (16 H)

4,10 s (2H) O-$CH_2$CO. 4,50-5,50 m (9 H), 6,50 d (1H) NH, 7,3 - 7,70 m (8 H) benzyl benzoate m.p, 8,10 d (2H) benzoate o.

l) - préparation de 7 méthoxycarbonyl 3,6 dioxaheptyl 2 acétamido 6-O benzoyl 4-O-/2,3,4,6 tétra-O-acétyl-β - D - galactopyranosyl/-2-désoxy-β-D-glucopyranoside (composé 13

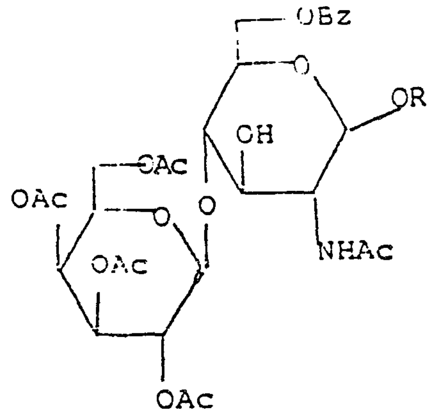

475,5 mg de produit de départ sont dissous dans 60 ml de méthanol anhydre. On ajoute environ 100 mg de Pd/C 10%. On soumet à agitation sous pression d'hydrogène pendant une nuit. La réaction est suivie en ccm ($CHCl_3$-Acétone 6/4). Après filtration et concentration, on récupère un sirop qui est chromatographié sur une colonne de gel de silice 30 g. ($CHCl_3$-Acétone 6/4).

On obtient 301,5 mg d'un sirop incolore.

Rendement 70,3% $[\alpha]_D^{20}$ =-0,43 (c = 4, $CHCl_3$).

Spectre de RMN ($CDCl_3$) : $\delta$ = 1,92 - 2,10-2,20. s (15H) N-Ac $CH_3CO$, 3,50 - 4,06 m. (13 H), 4,12-4,30 - m(4H), 4,30-5,5m (8H), 6,70d (1H) NH; 7.30-7,70 (m) 3H, 8,10d(2H) benzoate ortho.

m) - préparation du 1-O-(N-méthyl) acétimidyl 2,3,4 tri-O-benzyl-β-L-fucopyranoside (composé 14) de formule

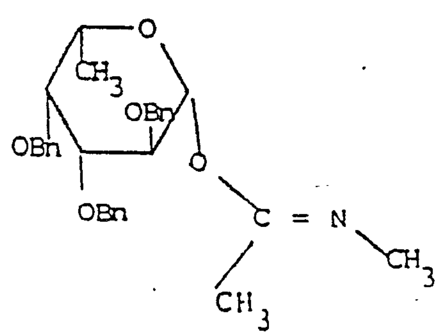

On prépare tout d'abord le réactif de Vilsmeier en mélangeant

$PCl_5$ (1,040 g; 5 m-moles), $CCl_4$ (10ml), DMF (0,365 ml) et du tribenzyl fucose (868 mg, 2 m-moles).

Dans un tricol sous azote avec agitation, on verse le $PCl_5$ finement broyé et le $CCl_4$ percolé sur $Al_2O_3$. On agite 5 mn sous azote. On ajoute ensuite avec une ampoule à brome le DMF distillé sur BaO. On observe l'apparition d'un précipité blanc. On laisse 10 mn sous azote puis on bouche. On filtre la solution en boîte à gants et on rince les cristaux au $CCl_4$. Le précipité est ajouté au tribenzyl-fucose dissous dans du dichlorométhane (20 ml). On agite 1/2 heure à l'abri de l'humidité (ccm benzène-éther: 13/1).

On extrait au chloroforme puis on lave la solution avec de l'eau, du bicarbonate de sodium puis de l'eau. La phase organique est séchée par du sulfate de sodium. On filtre puis on concentre. On obtient un sirop jaune.

On prépare l'imidate comme suit :

on agite sous azote à l'abri de la lumière le N-méthylacétamide (162 mg ; 2,2 m-moles), du tamis 4 Å en poudre et del'oxyde d'argent (1,16 g) dans 20 ml de benzène anhydre on ajoute le dérivé chloré (sirop) et 0,38 ml de diisopropyl-éthylamine. Après agitation pendant une nuit, on filtre sur Célite puis on élue avec 100 cc d'éther contenant 0,1% de triéthylamine. On évapore des solvants puis on effectue une coévaporation avec du benzène. On obtient 1,17 g d'un sirop. On cristallise dans l'hexane puis on ajoute une goutte de triéthylamine. Onrécupère 860 mg de produit (rendement 88%)

P.F. = 88-89°C. $[\alpha]_D^{20} = - 65°$ (c = 1,2, benzène).

n) - <u>préparation, par réaction des composés 13 et 14, du 7 méthoxy-carbonyl 3,6 dioxa heptyl 2 acétamido 6-O-benzoyl 3-O-[2,3,4 tri-O-benzyl-α-L-fucopyranosyl] 4-O-[2,3,4,6 tétra-O-acétyl β-D-galactopyranosyl] 2 désoxy-β-D-glucopyranoside (composé 15)</u> de formule

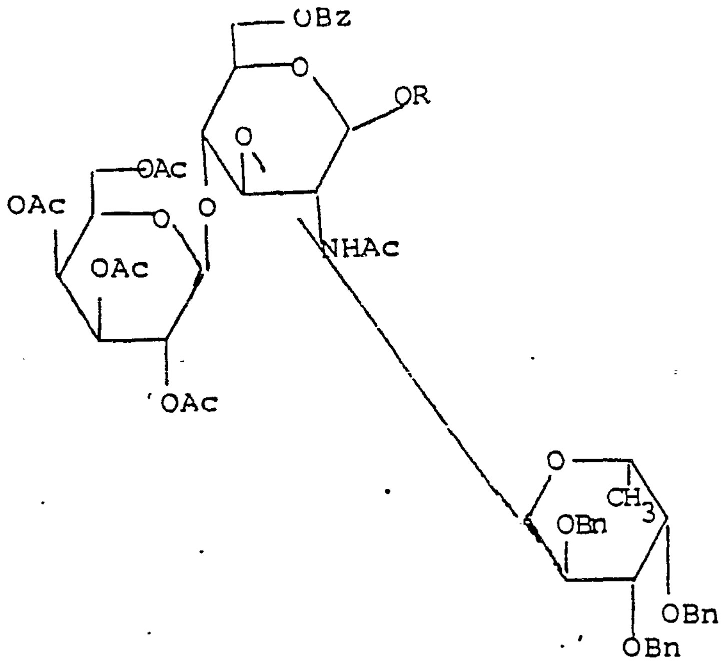

Sous argon on agite 100 mg d'aglycone (composé 13) dans 0,6 ml d'une solution d'acide paratoluène sulfonique (66,9 mg/ml dans du benzène) et du tamis moléculaire 4 Å en poudre.

Après une heure, on ajoute l'imidate (composé 14) (100 mg). Après agitation pendant une nuit, on ajoute 50 mg d'imidate. La réaction est suivie en ccm ($CHCl_3$ - acétone : 7/3). Après 8 heures, la solution est filtrée sur verre fritté et le tamis est rincé au dichlorométhane. On ajoute de la triéthylamine jusqu'à pH neutre puis on concentre. Le sirop obtenu est chromatographié sur une colonne de gel de silice (12 g) (éluant $CHCl_3$ - acétone ; 8/2). $[\alpha]_D^{20} = -32°$ ($c = 1,25$ ; $CHCl_3$). rendement de la condensation : 55 % -

o) - préparation du 7-méthoxycarbonyl 3,6 dioxaheptyl 2 acetamido 3-O-[2,3,4 tri-O-benzyl-α-L-fucopyranosyl] 4-O-[β-D-galactopyranosyl] 2 désoxy-β-D-glucopyranoside (composé 16).

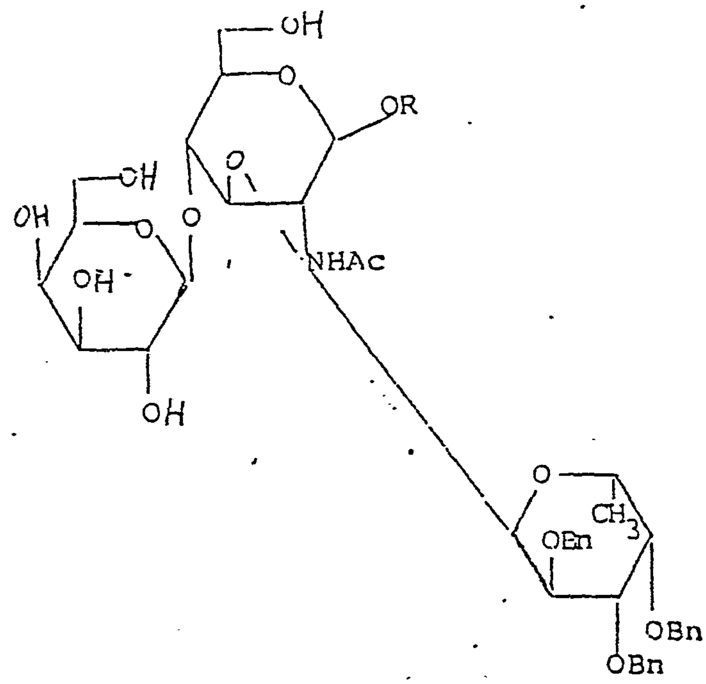

40,6 mg de produit de départ sont dissous dans 5 ml de méthanol anhydre. On ajoute quelques gouttes d'une solution de méthylate de sodium 2N dans le méthanol. On soumet à agitation à température ambiante pendant 30 heures. La réaction est suivie en ccm $CHCl_3$-MeOH: 6/4. Après neutralisation avec une résine Dowex H+ sèche, la solution est filtrée et concentrée. Le sirop obtenu est chromatographié sur une colonne de gel de silice (2 g). On utilise un mélange $CHCl_3$-MeOH (6/4) comme éluant. Rendement : 90-%

$[\alpha]_D^{20} = -76,4$ (C = 1,02 ; MeOH).

On récupère 28,7 mg d'une fraction pure. $[\alpha]_D^{20} = -76,4$ (c=1,02, MeOH

Rendement : 90%.

p) - préparation du composé 17

On dissout 28,7 mg du composé 16 dans 7 ml de méthanol anhydre. On ajoute environ 20 mg Pd/C à 10%. On agite sous pression d'hydrogène durant environ 14 h. La réaction est suivie en ccm (methanol-acétate d'éthyle-eau : 5/4/1). On filtre sur verre fritté puis on concentre la solution. On obtient le produit homogène en ccm. Rendement quantitatif. $[\alpha]_D^{20} = -58,2°$ (c = 0,78, méthanol).

EXEMPLE 5

Essais relatifs à la reconnaissance spécifique de la séquence 3-fucosyl-lactosamine.

Les résultats des essais a) et b) sont rapportés ci-après.

Dans les essais a), on a étudié l'inhibition de la fixation d'anti-SSEA-1 à du méconium d'origine humaine marqué par $I^{125}$, par

- d'une part, divers oligosaccharides naturels (c'est-à-dire constituant les chaînes de produits naturels),
- d'autre part, la 3-fucosyl-lactosamine elle-même, obtenue par voie de synthèse et un tetrasaccharide de synthèse.

Les essais b) se rapportent à l'étude de l'inhibition de la fixation d'anti-SSEA-1 à du méconium marqué par $I^{125}$ par

- du méconium,
- du placenta,
- et du fluide amniotique,

tous trois d'origine humaine.

Ces essais sont réalisés selon les techniques radio-immunologiques, avec utilisation de deux anticorps. Le procédé de détermination utilisé correspond à une méthode modifiée par rapport à celle décrite par Wood, E., Lecomte, J., Childs, R.A. & FEIZI, T. Mol. Immunol. 16, 813-819 (1979)

L'anticorps anti-SSEA-1 (dilution 1 : 3000 dans de la sérum albumine bovine, tampon phosphate) est obtenu à partir d'un liquide d'ascite de souris BALB/c traitée avec du prispane, et à laquelle on a injecté des cellules hybrides produisant des anticorps.

A titre de véhicule, on utilise du sérum normal de souris (dilution 1 : 100 ).

Le deuxième anti-corps est constitué par un sérum anti-souris d'immunoglobuline de lapin, non dilué (de Dako-immunoglobulins, Copenhague, Danemark).

Essais a

- Les oligosaccharides utilisés sont les suivants (on indique pour chacun d'eux le symbole utilisé sur les courbes données sur la figure 1, leur structure et leur désignation).

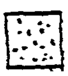 Galβ1 → 4GlcNAcβ1 → 6?- 3 hexenetetrol(s) N-1 $R_L$0.71a
↑ 1,3
Fucα

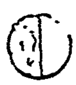 Galβ1 → 3GlcNAcβ1 → 3 ou 4 galactitol N-1 $R_L$0.28
↑ 1,4
Fuc α

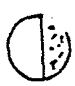 Galβ1 → 3GlcNAcβ1 → 3Galβ1 → 4Glc Lacto-N-difucohexaose II
↑ 1,4 ↑ 1,3
Fucα Fuc α

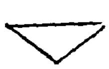 Galβ1 → 3GlcNAcβ1 → 3Galβ1 → 4Glc Lacto-N-fucopentaose II (LNFP II)
↑ 1,4
Fuc α

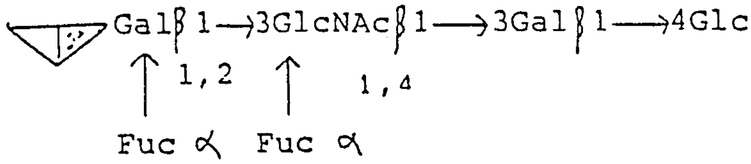

Lacto-N-difucohexaose I (Le^b)

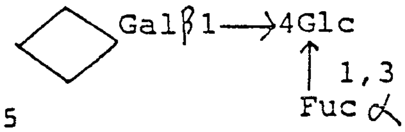

3-fucosyl lactose

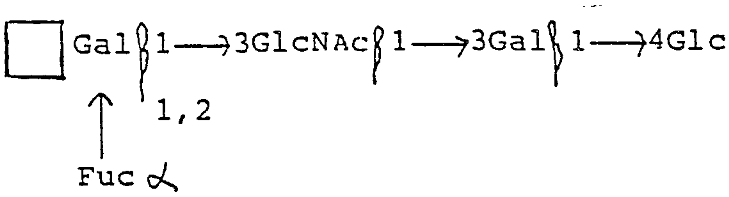

Lacto-N-fucopentaose I (H)

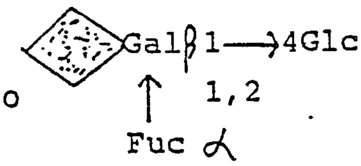

2 fucosyl lactose

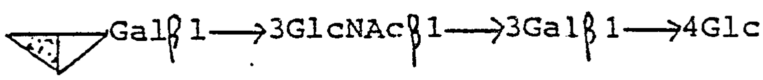

Lacto-N-tétraose

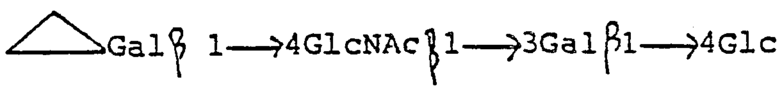

Lacto-N-néotétraose

Il s'agit d'oligosaccharides avec des séquences (Galβ1→3GlcNAc), dites de type 1 ou (Galβ1→4GlcNAc/Glc) dites de type 2.

Les résultats de ces essais d'inhibition sont rapportés sur la fig. 1.

Il apparaît que les oligosaccharides avec des séquences de type 1 sont inactifs en tant qu'inhibiteurs de l'anti-SSEA-1.

En revanche, une inhibition très importante apparaît avec l'oligosaccharide appelé N-1 $R_L$ 0,71a qui contient une séquence fucosylée de type 2.

En effet, 0,5nmole donne une inhibition de 50%.

Cet oligosaccharide a été isolé à partir d'une glycoprotéine N-1, après dégradation alcaline partielle (Lloyd, K.O., Kabat, E.A. & Licerio, E. Biochemistry, 7, 2976-2990 (1968)).

Il est intéressant de remarquer la faible inhibition obtenue avec l'oligosaccharide renfermant la séquence 3- fucosyl-lactose , ce qui indique l'importance du groupe N-acétyl-glucosamine dans la fonction du SSEA-1 en tant que déterminant antigénique.

Les oligosaccharides actifs Le$^a$ (Lewis$^a$) possédant une structure de type 1 avec séquence terminale

Gal β 1 → 3 GlcNAc

↑ 1,4

Fuc α

se sont révélés au moins 10 fois moins actifs que leurs isomères de type 2. Il a été montré du reste que cette activité étant d'ailleurs due à la présence des isomères de type 2 (on notera qu'il s'agit, en effet, de produits d'origine naturelle qui ne sont donc pas purs). Le lacto-N-difucohexose I (Le$^b$ actif), le lacto-N-fucopentose I (H actif) et le 3-fucosyl-lactose sont apparus 200 à 1000 fois moins actifs en tant qu'inhibiteurs.

Le 2-fucosyl-lactose, le lacto-N-tétraose et le lacto-N-neotétraose se sont révélés inactifs.

L'ensemble de ces résultats montre bien la reconnaissance spécifique par l'anti-SSEA-1 de la structure 3-fucosyl-N-acétyl lactosamine comme déterminant antigénique.

Une confirmation de ces résultats est donnée en étudiant l'inhibition obtenue avec la 3-fucosyl-lactosamine préparée selon le procédé de JACQUINET et SINAY évoqué ci-dessus et le tétrasaccharideO-α-L-Fucopyranosyl-(1 → 2)-O-β-D-galactopyranosyl-(1 → 4)-O- [α-L-fucopyranosyl-(1 → 3)]-2-acétamido-2-déoxy-α-D-glucopyranose

Gal β 1 → 4 Glc NAc
↑ α 1,2 ↑ α 1,3
Fuc Fuc

préparé selon JACQUINET et SINAY, J.O.C. 42,720,1977.

Les résultats de l'inhibition observée sont rapportés sur la fig. 2 (courbe avec X) dont l'examen montre une inhibition complète de la fixation, alors qu'aucun effet n'est observé avec le tétrasaccharide de synthèse

Ces résultats établissent clairement la reconnaissance en tant qu'antigène de la séquence trisaccharidique en question.

Essais b

En opérant comme précédemment, on a utilisé comme substances inhibitrices du placenta, du méconium et du fluide amniotique d'origine humaine.

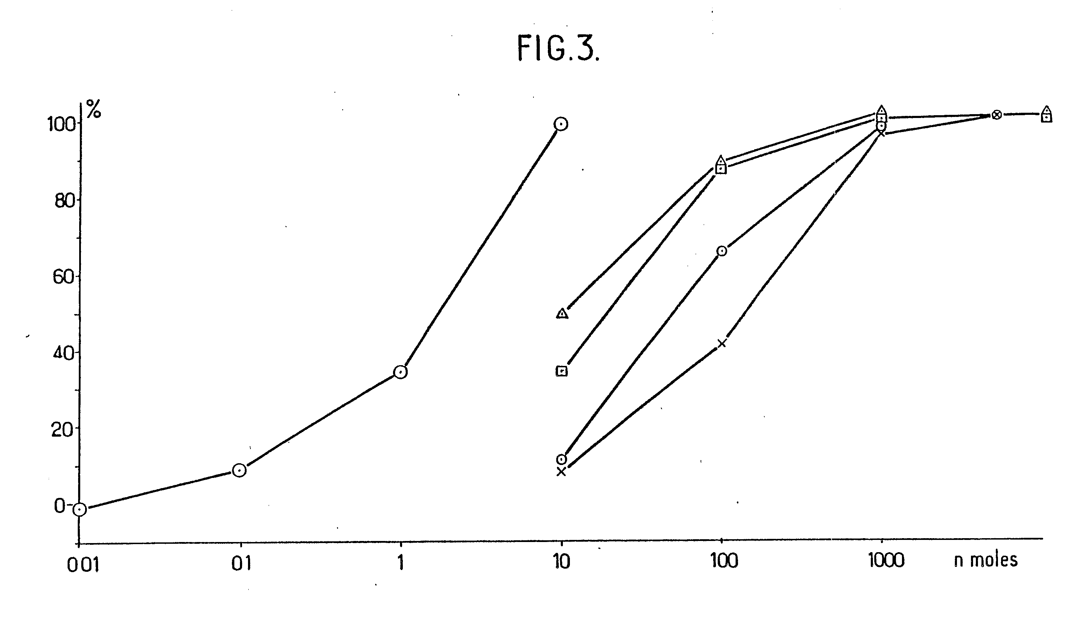

Les résultats d'inhibition obtenus sont rapportés sur la fig. 3 où le symbole ⊗ se rapporte au méconium X et ⊙ à deux essais sur du placenta, ⚠ et ⊡ à deux essais sur du fluide amniotique.

Il ressort de ces résultats que la séquence 3-fucosyl lactosamine s'exprime dans les substances étudiées où elle peut donc être avantageusement mise en évidence avec les réactifs de l'invention aux fins des diagnostics des développements de grossesses.

EXEMPLE 6 : Préparation d'un antigène artificiel à l'aide du trisaccharide de l'exemple 2 et utilisation comme réactif de diagnostic.

Selon des méthodes en soi connues (activation, puis fixation), on a fixé, dans différents essais, le trisaccharide de l'exemple 2, comportant une chaîne de substitution à terminaison amino, respectivement sur des billes de latex de verre et d'agarose.

L'antigène fixé, ainsi préparé, a été mis en contact avec le sérum à tester. On a, de cette façon, obtenu une réaction d'agglutination, ce qui a permis la détection de la présence d'anticorps dans le sérum.

Ce test est particulièrement intéressant pour reconnaître la présence d'anticorps anti-3-fucosyl N-acétyl lactosamine dans le sérum de la femme enceinte.

EXEMPLE 7 : Elaboration d'anticorps spécifiques à l'aide du trisaccharide de l'exemple 3 et application pour la détection de tératomes.

On a procédé selon des techniques connues à la fixation du trisaccharide de l'exemple 3 sur différents véhicules porteurs, à savoir l'albumine, l'albumine de sérum bovin, la thyrogluboline, la polylysine et la poly-L-alanine-lysine qui sont des supports compatibles avec l'administration à l'animal.

L'antigène ainsi fixé a été administré au lapin et on a recueilli un immunsérum que l'on a purifié pour obtenir les immunoglubolines spécifiques.

Cet anticorps spécifique a été fixé sur billes de latex (dans d'autres essais, on a utilisé des billes de verre ou d'agarose) et a permis l'identification par réaction d'agglutination de l'antigène 3-fucosyl-N-acétyl lactosamine dans un tératome.

EXEMPLE 8 : En utilisant le dérivé de l'exemple 4, fixé sur de la polylysine, et en formant des anticorps vis-à-vis du réactif antigénique, on a également détecté une tumeur gastro-intestinale.

On remarquera à cet égard que cette séquence 3-fucosyl-N-acétyl-lactosamine constitue le déterminant antigénique de différents types de tumeurs, qui sont donc détectables grâce aux moyens fournis par l'invention.

Ces anticorps formés comme décrit ci-dessus et marqués avec un marqueur spécifique ou non, compatible en vue d'une administration à l'homme (par exemple $I^{131}$, $Tc^{99}$) peuvent être utilisés pour des détections in vivo et des localisations de tumeurs.

Ces anticorps peuvent être aussi utilisés en médecine pour des théraphies anti-tumeurs. On peut les préparer sous forme de préparations pharmaceutiques, en particulier sous forme de préparations injectables. On peut les mettre en oeuvre tels quels pour une administration intra ou péri-tumorale ou les encapsuler dans des liposomes, ou encore les utiliser comme substances hôtes associées ou conjuguées à des produits anti-tumoraux ou de toute autre façon connue de l'homme de l'art.

REVENDICATIONS

0069678

1. Trisaccharides dérivés du O-α-L-fucopyranosyl-(1⟶3)-[O-β-D-galactopyranosyl-(1⟶4)-2-acétamido-2-déoxy-D-glucopyranose] ou 3 fucosyl-N-acétyl-lactosamine, caractérisés en ce qu'ils répondent à la formule :

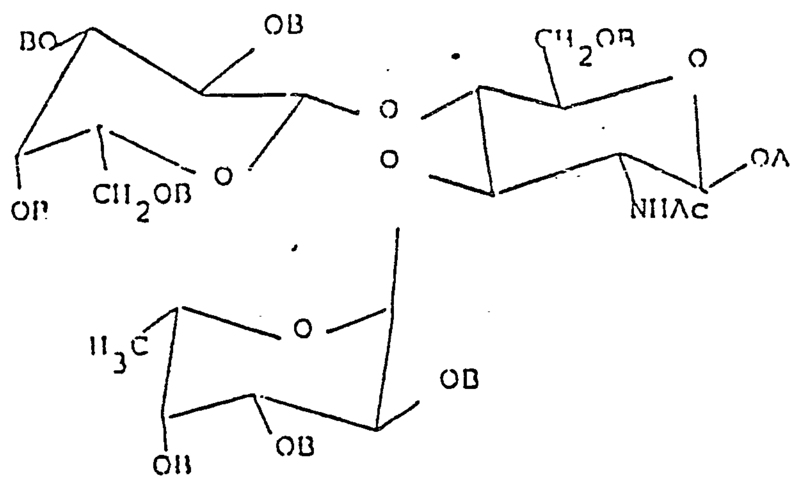

dans laquelle :

A représente

- un groupe alcoyle comportant 2 à 10 atomes de carbone, ou
- un groupe hydroxy-alcoyle avec 2 à 10 atomes de carbone comportant un ou plusieurs groupes hydroxyle, avantageusement un groupe hydroxyle en bout de chaîne, ou
- un groupe comportant une ou plusieurs liaisons insaturées, en particulier au moins un radical éthyléniquement insaturé, choisi parmi les radicaux alcényle, et plus spécialement les radicaux alcényle à 2 à 10 atomes de carbone, ou encore
- l'un des groupes précédents, mais comportant un ou plusieurs groupes éther, notamment deux ou encore trois et/ou amine intercalaires,
- les différents groupes représentés par A pouvant être terminés par un groupement azoté notamment amino, amido, hydrazine, azido, ou un groupement acide carboxylique éventuellement éthérifié ou estérifié .
- les substituants B, identiques ou différents les uns des autres représentent, le cas échéant avec un substituant voisin des groupes de protection de radicaux hydroxyle et sont choisis parmi des groupes stables, non réactifs dans les conditions habituelles de synthèse osidique et facilement éliminables dans des conditions douces, compatibles

avec le maintien de la structure osidique, en particulier, par des groupes benzyle, benzoyle, benzylidényle ou acétyle ou représentent un atome d'hydrogène.

2. Trisaccharides selon la revendication 1, caractérisés en ce que A représente un radical alcoyle comportant 2 à 10 atomes de carbone, substitué par au moins un groupe hydroxyle, avantageusement en bout de chaîne, notamment un radical alcoylèneglycol ou hydroxyalcoyle, ou encore un radical carboxylique ou un dérivé d'un tel groupe tel qu'un éther ou un ester.

3. Trisaccharides selon la revendication 1, caractérisés en ce que A répond aux structures (1), (2) ou (3) suivantes :

(1) : $-(CH_2)_n - O - (CH_2)_{n'} - O - (CH_2)_m - R$

(2) : $-(CH_2)_{n''} - O - (CH_2)_{m'} - R$

(3) : $-(CH_2)_{n'''} - R$

dans lesquelles n, n' et m, identiques ou différents les uns des autres, sont égaux à 1, 2, 3, 4 ou 5, m pouvant en outre être égal à 0, la somme de n + n' et éventuellement m étant avantageusement un nombre de 4 à 10, de préférence égal à 6, n" et m', identiques ou différents l'un de l'autre, sont égaux à 1, 2, 3, 4 ou 5, m' pouvant être égal en outre à 0, n" ou éventuellement n" + m' étant avantageusement un nombre de 4 à 10, de préférence égal à 6 ou 8, et n''' est un nombre de 1 à 10, avantageusement de 4 à 10, de préférence égal à 8, et R représente un groupement fonctionnel.

4. Trisaccharides selon la revendication 3, caractérisés en ce qu'ils comportent une chaîne A de structure (1) ou (2), dans laquelle R représente un atome d'hydrogène.

5. Trisaccharides selon la revendication 3, caractérisés en ce que A représente des chaînes possédant la structure (1), (2) ou (3), dans laquelle R représente un groupe -COOR' ou -COR', R' représentant un atome d'hydrogène ou un radical alcoyle avec 1, 2 ou 3 atomes de carbone ou encore R représente un groupe $-CONH_2$.

6. Trisaccharides selon la revendication 3, caractérisés en ce que R représente un groupe azoté $-NH_2$, $-N_3$ ou $-NH-NH_2$.

7. Trisaccharides selon la revendication 3, dans lesquels A présente la structure (1) ou (2), dans laquelle R représente un groupe alcényle avec 2 à 10 atomes de carbone, de préférence un groupe allyle.

8. Trisaccharides caractérisés en ce qu'il s'agit du :

8-azido-3,6-dioxa octyl 2-acétamido-3-O-[α-L-fucopyranosyl]-4-O[β-D-galactopyranosyl]-2-désoxy-β-D-glucopyranoside ;

8-amino-3,6-dioxa octyl-2-acétamido-3-O-[α-L-fucopyranosyl]-4-O-[β-D-galactopyranosyl]-2-désoxy-β-D-glucopyranoside ;

8-méthoxy carbonyloctyl 3,6-dioxa 2-acétamido 3-O [α-L-fucopyranosyl]-4-O[β-D-galactopyranosyl]-2-désoxy-β-D-glucopyranoside ;

7-méthoxy carbonyl 3,6 dioxa heptyl 2 acétamido 3-O[α-L-fucopyranosyl] 4-O[β D-galactopyranosyl] 2 désoxy-β-D-glucopyranoside .

9. Procédé de préparation de trisaccharides répondant à la structure donnée dans la revendication 1, selon une technique générale comportant la réaction d'un dérivé réactif de galactopyranosyle avec un dérivé réactif de glucosamine et la condensation du disaccharide correspondant avec un dérivé réactif de fucopyranosyle, caractérisé en ce que pour l'élaboration de trisaccharides dans lesquels A représente plus spécialement une chaîne de type (1), on met en oeuvre un dérivé réactif de glucosamine soumis auparavant à une réaction de condensation avec du diéthylèneglycol ou du triéthylène glycol, ou pour l'élaboration de trisaccharides dans lesquels A représente plus spécialement une chaîne de type (3) on met en oeuvre un dérivé réactif de glucosamine tel qu'une 1,2-oxaline, avec un acide monocarboxylique de structure :

$HO-(CH_2)_{n'''}-COOR$

dans laquelle n''' et R présentent les significations données dans la revendication 3, ou pour l'élaboration de trisaccharides dans lesquelles A représente un groupe allyle ou une chaîne pouvant être obtenue en utilisant la réactivité du groupe allyle, on procède tout d'abord à la synthèse d'un trisaccharide répondant à la structure des trisaccharides de l'invention, dans lequel tous les groupes -OH sont engagés dans des groupements protecteurs, et sont avantageusement benzylés, selon les réactions classiques de la synthèse organique, on introduit un groupement allyle en position 1, en β, de la glucosamine, et l'on procède, si on le désire, à l'allongement de la chaîne, selon lesréactions classiques de synthèse, de manière à introduire les groupements chimiques souhaités, notamment on soumet le dérivé allyle à une réaction d'hydroboration, en utilisant un réactif tel que le 9-borabicyclo /̄3.3.1./ nonane, puis à l'action d'une base, ce qui conduit à une chaîne terminée par un groupement fonctionnel alcool, on éthérifie si on le désire ce groupe ou on le traite par un halogénure d'allyle pour introduire un nouveau groupement allyle qui sera à son tour avantageusement soumis à une réaction d'hydroboration, ce dérivé étant à son tour, si on le désire, soumis à une réaction de tosylation et le dérivé tosylé obtenu à une réaction avec un azide, conduisant à une chaîne A à terminaison azido.

10. Réactifs immunologiques possédant la structure 3-fucosyl N-acétyl lactosamine et constitués par les trisaccharides selon l'une quelconque des revendications 1 à ou le trisaccharide 3-fucosyl-N-acétyl lactosamine, se présentant tels quels, ou sous forme immobilisée, notamment pour l'élaboration de réactifs de diagnostics, antigènes artificiels ou immunoabsorbants.

11. Anticorps spécifiques vis-à-vis du trisaccharide 3-fucosyl-N-acétyl-lactosamine et de ses dérivés de substitution selon l'une quelconque des revendications 1 à 8.

FIG.1.

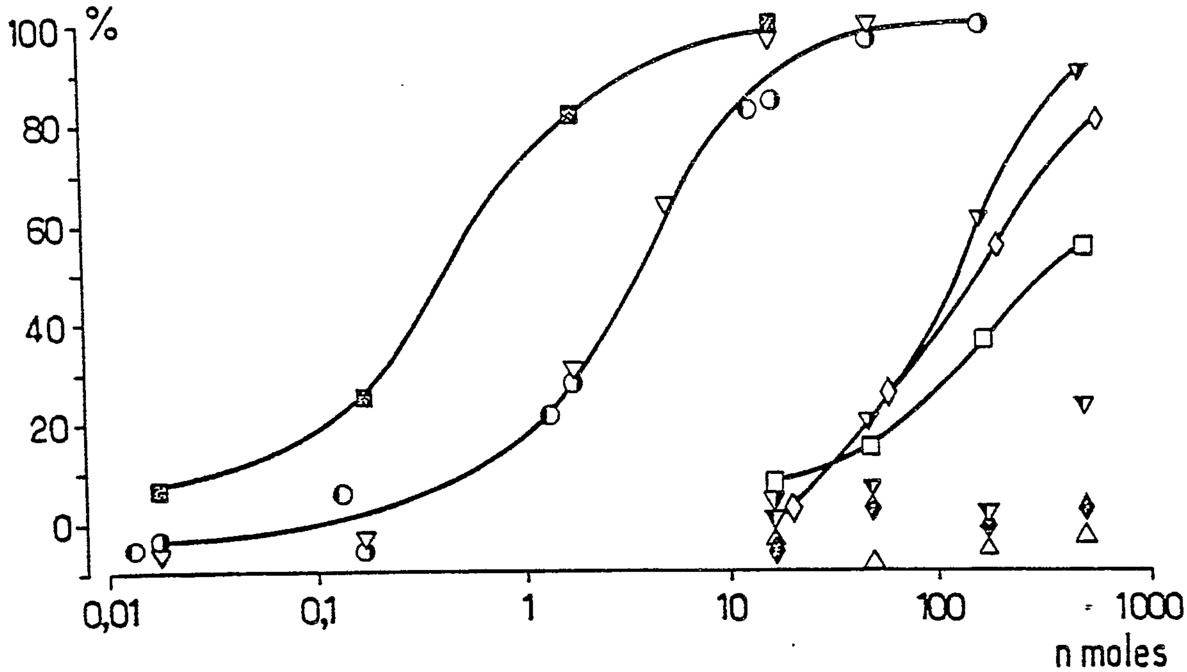
%
100
80
60
40
20
0
0,01
0,1
1
10
100
1000
n moles

FIG.2.

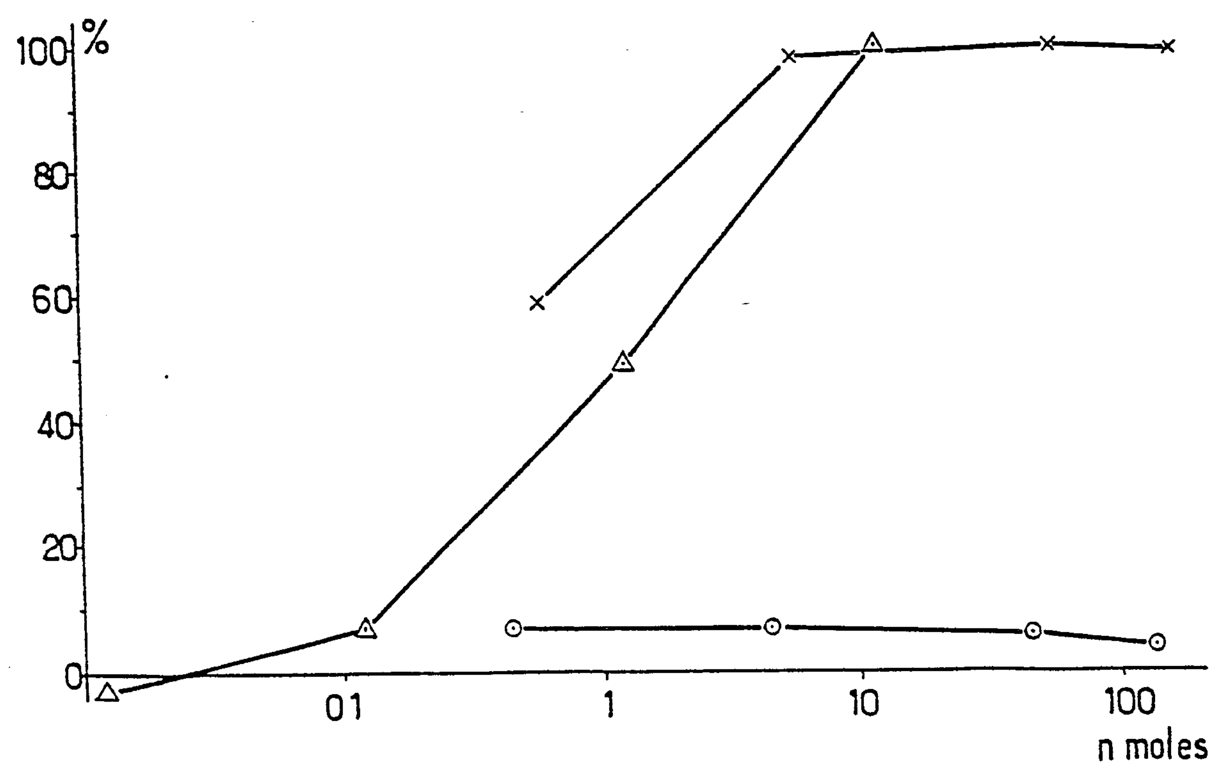
%
100
80
60
40
20
0
01
1
10
100
n moles

2/2 0069678

FIG. 3.